# EUROPEAN PATENT SPECIFICATION

(11) **EP 1 796 534 B1**
(45) Date of publication and mention of the grant of the patent: **19.11.2008**
(21) Application number: 04762771.6
(22) Date of filing: 19.08.2004
(51) Int. Cl.: A61B 5/00

(54) **MICRO-ANALYSIS SYSTEM**
VORRICHTUNG ZUR DURCHFÜHRUNG VON MIKROANALYSEN
SYSTÈME MICROANALYTIQUE

(43) Date of publication of application: 20.06.2007
(73) Proprietor: Danfoss A/S, 6430 Nordborg (DK)
(72) Inventor: ARNDT, Heiko, D-24937 Flensburg (DE)
(86) International application number: PCT/DK2004/000544
(87) International publication number: WO 2006/018022

(56) References cited:
- EP-A- 0 534 074
- US-A- 5 640 954
- US-A- 6 091 976
- US-A1- 2002 082 490

## Description

The invention relates to a micro-analysis system for determining the concentration of a species in a medium and more specifically for continuous monitoring of the concentration of a species in vascular body fluid of a human being.

Reliable knowledge of the concentration of certain species, e.g. the glucose concentration in the blood of a patient, is essential for medical treatments to ensure adequate dosage of medication. Diabetic patients need supply of insulin medication corresponding to the varying glucose concentration, which may vary significantly over time, especially in connection with the intake of food.

Widely used is a method to take a plurality of blood samples during a day, typically by penetrating the skin of the patient with needles or cannulas, and to individually determine the glucose concentration on the basis of this samples. This method presents certain discomforts for the patient.

It is further known to permanently implant subcutaneous sensing probes designed to collect glucose molecules from the vascular body fluid. Such sensing probes may periodically or continuously be connected to an external analysis system. With this method, a carrier fluid, typically a saline solution is pumped from a system reservoir through the sensing probe. Suitable collecting means in the sensing probe allow transfer of glucose molecules into the carrier fluid. This species enriched carrier fluid, normally referred as sample fluid, is then returned to the analysis system, where the concentration of glucose in the sample fluid, which correlates with the concentration in the body of the patient, is determined. In known methods, at least one reagent fluid is mixed with the sample fluid received from the sensing probe to obtain a reaction product, which is suitable for proper generation of a measuring signal in a detector of the analysis system.

Since the carrier fluid has to stay a certain amount of time within the probe to allow significant transfer of species from the body fluid into the carrier fluid, the carrier fluid flow is very low. Typical flow rates are in the range of a few µl/min, which are realized by flow restrictions being arranged in the fluid channels. When connecting an analysis system to the sensing probe, the flow channel from the carrier fluid reservoir of the analysis-system to the probe and the probe volume itself have to be completely filled before monitoring of species concentration can be performed. This time period for priming the system can be of considerable length, typically one hour.

Document US 6,091,976 discloses a system as defined by the preamble of claim 1

It is therefore an object of the invention to provide a micro-analysis system with considerably reduced priming time.

This is realized by an analysis system according to claim 1, comprising
- storage means in the form of at least one first fluid reservoir holding reagent fluid and at least one second fluid reservoir holding carrier fluid,
- sensing means for collecting species from the medium, said sensing means having an inlet and an outlet,
- analysing means for determining the concentration of the species in the medium,
- connecting means, comprising first connecting means for fluid connection between the first fluid reservoir and the analysing means, second connecting means for fluid connection between the second fluid reservoir and the inlet of the sensing means and third connecting means for fluid connection between the outlet of the sensing means and the analysing means,
- said first connecting means comprising at least one first flow restricting means and said second connecting means comprising at least one second flow restricting means,
where a third fluid reservoir holding priming fluid and fourth connecting means for connection between said third fluid reservoir and the inlet of the sensing means are provided.

Fluid flow from this third reservoir, typically containing the same carrier fluid as the second reservoir and being connected directly to the sensing means, is not subjected to the flow restrictions arranged within the flow connections between carrier fluid reservoir and sensing means. Hereby, a higher fluid flow in the fourth connecting means and a much faster priming of both the sensing means and the flow channels between its inlet and the carrier fluid reservoir is achieved.

Preferably, said fourth connecting means comprise at least one third flow restricting means.
A certain limiting of the priming fluid flow and of the fluid pressure within the sensing means is necessary to protect the sensing means, e.g. a sensing probe, from being destroyed. To increase the transfer rate of species from the medium to the carrier fluid flowing through the sensor probe, a part of the sensor wall, which represents the interface between medium and carrier fluid, has to be made very thin. A low restriction within the priming fluid connecting means leads to an adequate pressure drop.

Preferably, this third flow restricting means within the priming fluid connection means exhibits less flow restriction than second flow restricting means within the carrier fluid flow channel.
Hereby, an increased priming fluid flow in relation to the carrier fluid flow during normal operation of the analysis system is achieved. However, the third flow restriction is designed to assure sufficient pressure drop over the restriction to limit the pressure within the sensing means.

Preferably, the volume of the third fluid reservoir is substantially equal to the total volume of said second and fourth connecting means and of said sensing means.
By this feature, the volume of the priming fluid reservoir is adapted to the volume of the system, which has to be filled up, before collecting of species from the medium can start. No additional space for a larger priming fluid reservoir is required. When all the fluid connections from the analysis system towards the sensing means and the sensing means itself are filled up, carrier fluid reservoir will take over to provide fluid flow towards the sensor. The system will be ready to detect and monitor species concentration as soon as sample fluid from the sensor reaches the analysis means.

Preferably, said fourth connecting means communicate with said second connecting means downstream said second and third flow restricting means.
Instead of providing a separate priming fluid flow channel all the way from the priming fluid reservoir to the sensing element, the priming fluid, after having passed the flow restriction within the priming fluid channel, enters the carrier fluid channel at a position downstream the flow restriction within that channel. This simplifies the design and manufacturing of the system, and minimizes the volume of the priming fluid channel.

Preferably, the micro-analysis system further comprises means for pressurizing said fluid reservoirs. Hereby, the flows of fluid within the system may - in addition to the flow restrictions -be controlled by the pressure exerted on the fluid reservoirs. If required, individual flow control and adaptation is possible, e.g. for reagent fluids or the priming fluid.

Preferably, at least parts of the walls of the fluid reservoirs are flexible. Fluid flow from reservoirs with flexible walls, e.g. in the form of flexible bags, can easily be controlled by exerting a force on the outside of the flexible wall parts. Suitable pressurizing means may be elastic bandages surrounding the reservoirs.

Preferably, the storage means further comprise a storage container with a fluid inlet in communicating with said pressurizing means, said storage container delimiting a fluid chamber, within which the fluid reservoirs are arranged. In this embodiment, all fluid reservoirs are stored in a common container having a fluid chamber in fluid communication with the pressurizing means. When the reservoirs are being emptied successively during operation of the system, the volume of the reservoirs will decrease, while at the same time the volume of the fluid chamber will increase correspondingly.

Preferably, the pressurizing means is a constant pressure source. With a constant pressure acting on the fluid reservoirs, a relatively simple and safe control of the amount of fluid delivered from the fluid reservoirs is achieved.

Preferably, said storage means, said connecting means, said analysing means and said pressurizing means are arranged within a common housing. Integration of all parts of the micro-analysis system, except the sensing means, in a single housing provides a compact, robust system unit, which only has to be coupled to the inlet and outlet of the sensing element. When used in medical applications, e.g. for continuous monitoring of species in a human body, the patient may carry the system unit directly on his body in a discreet and comfortable manner.

In case of a subcutaneous sensing probe with its inlet and outlet arranged on the outer side of the skin, a very easy replacement of the analysis system unit is possible, e.g. if the storage means are emptied.

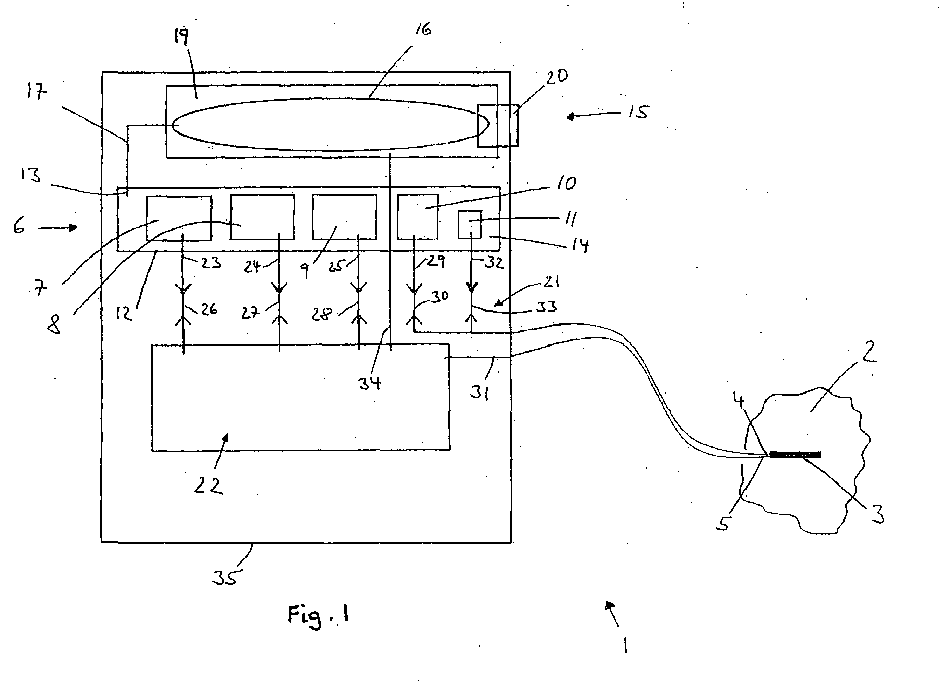

A preferred embodiment of the invention will in the following be described with reference to the drawing, where Fig.1 shows a schematic diagram of a micro-analysis system.

Fig.1 shows a micro-analysis system 1 for determining or monitoring the concentration of a species in a medium 2, e.g. in a vascular body fluid of a human being.
With such systems, the glucose concentration in the blood of a patient may be monitored. The analysis results of the system may be used to determine the right moment and the adequate amount of insulin to be applied to the patient for ensuring well-being of the patient.

The system comprises a sensor probe 3, having an inlet 4 and an outlet 5, for collecting the species from the medium. The sensor probe may be implanted subcutaneously in the body of the patient. Alternatively, the sensor probe may be exposed to a body fluid sample taken from the patient prior to analysing. The sensor probe is designed to collect species, e.g. glucose molecules, from the body fluid and to transfer the species into the carrier fluid. Suitable probes are known in the art, and may, as an example, take advantage of molecular diffusion effects through semipermeable membranes caused by concentration gradients for the species between both sides of the membrane.

The system 1 further comprises storage means 6 with first fluid reservoirs 7, 8 ,9 containing reagent fluid, a second fluid reservoir 10 containing carrier fluid, and a third fluid reservoir 11 containing priming fluid. All fluid reservoirs are arranged within a storage container 12 having an inlet 13 and delimiting a fluid chamber 14. The reservoirs are formed as flexible bags.
As both carrier and priming fluid may be used an identical innocuous fluid, e.g. saline solution.
Pressurizing means 15 comprise an elastomeric bladder 16 containing pressurized fluid, e.g. saline solution, the bladder being in communication with the fluid chamber 14 in the storage container 12 via connecting line
17 and storage container inlet 13. Fluid chamber 14 is hereby filled with pressurized fluid, which exerts a constant force on the fluid reservoirs 7, 8, 9, 10 and 11. The elastomeric bladder 16 acts hereby as constant pressure source, simultaneously acting on for the fluid reservoirs via fluid chamber 14. Other embodiments of pressurizing means, as individual elastomeric bandages surrounding each fluid reservoir, are of course possible.
With the shown embodiment however, all the reservoirs 7, 8, 9, 10 and 11 are exposed to the same constant pressure, which simplifies the control of amount of fluid delivered from the reservoirs.

Elastomeric bladder 16 is arranged within a protective container 18, which encloses a chamber 19 delimited by the elastomeric bladder and the inner walls of container 18. A self-sealing inlet arrangement 20, e.g. a commonly used elastomeric membrane, allows access from the environment to the elastomeric bladder 16 and is designed to fill or refill of the latter with pressurizing fluid, e.g. by use of a syringe.

System 1 further comprises connecting means 21 for communicating fluid between the reagent fluid reservoirs 7, 8, 9 and analysing means 22, and between carrier and priming fluid reservoirs 10,11 and sensor probe 3. First connecting means in the form of channels 23, 24, 25 are designed to deliver reagent fluids to the analysing means, the flow rate being controlled by first flow restrictions 26, 27 and 28 arranged in channels 23, 24 and 25. The flow restrictions allow individual control of reagent flow towards the analysing means 22, necessary to perform the desired actions within analysing means 22.
A channel 29 represents second connecting means and communicates carrier fluid reservoir 10 with the inlet 4 of sensor probe 3, the flow rate in channel 29 being controlled by second flow restriction 30. Third connecting means, e.g. in the form of a channel 31, allow return of carrier fluid enriched with the species collected by the probe 3, also called sample fluid, back to the analysing means 22. Here, sample fluid is exposed to mixing with the reagent fluids from reservoirs 7, 8, 9, resulting in certain chemical reactions, which can facilitate the detection and measurement of species concentrations in a suitable detector arrangement known in the art.

To improve comfort for the patients, especially in case of implanted sensor probes, great efforts are done to minimize the volume of both the probes and the analysing system. The same applies in regard of cost savings by minimizing the amount of reagent fluids and simplifying the design of the overall analysis-system.
At the same time, the diffusion rate of species through the sensor membrane is relatively low.
This leads to very low flow rates, necessary to achieve sufficient concentration levels of species in the sample. If the concentrations are too low, accuracy of determination of the species concentration in the medium is insufficient. Tests have shown, that minimum flow rates of 0.3 µl/min, controlled by flow restriction 30 are necessary to obtain required accuracy. The total volume of the sensor probe 3 and the channel 29 between carrier fluid reservoir and sensor probe, also called dead volume, is typically in the range of 10 to 20 µl. When initially connecting the sensor probe to the analysing system and pressurizing the elastomeric bladder 16, channel 29 and probe 3 have to be filled up with carrier fluid, and the resulting sample fluid has to return to the analysing means through channel 31, before monitoring of species concentration is enabled. This so-called priming time may be as long as one hour.

To reduce this priming period, the priming fluid reservoir 11 is connected to the inlet 4 of sensor probe 3 through fourth connecting means 32 with third flow restricting means 33. Flow restriction means 33 allow a much higher fluid flow than restriction means 30, whereby priming time can be reduced as much as to a few minutes. Further reduction is hereby limited by the fluid pressure allowed in sensor probe to avoid damage of the latter, e.g. by rupture of the membrane.
Channel 32 opens into channel 29 downstream of flow restriction 30.

To minimize the total size of the analysis-system, the volume of priming fluid reservoir 11 is adapted to the dead volume of the system. When fluid chamber 14 is pressurized by filling up of elastomeric bladder 16, priming fluid will be delivered from reservoir 11, due to the lower degree of flow restriction in channel 32. When reservoir 11 is emptied, channel 29 and sensor probe 3 are completely filled and carrier fluid reservoir 10 will "take over" to deliver carrier fluid at nominal flow rate.

A waste fluid channel 34 is provided to communicate analysing means 22 with chamber 19 inside protective container 18. During operation of the system, the volume of elastomeric bladder 16 will decrease, as pressurizing fluid continuously is displaced into fluid chamber 14 of storage container 12. The resulting free space in chamber 19 can thus be used as waste reservoir. Hereby, a self-contained system is achieved, which can be disposed after end of use, when all the reagent and carrier fluid reservoirs are emptied.

The entire analysis-system, except sensor probe 3 and corresponding connecting means 29 and 31, is enclosed in a sealed housing 35. A diabetes patient with implanted sensor probe is enabled to constantly monitor the glucose concentration. The miniaturized single-use system can easily be replaced after end of use by disconnecting the housing 35 from the sensor probe, reconnecting a new system and initialising by filling up the elastomeric bladder. Due to the new priming arrangement, the interruption of monitoring of the glucose concentration is minimized.

## Claims

1. Micro-analysis system for determining the concentration of a species within a medium, said system comprising
- storage means (6) for the storage of fluids, comprising at least one first fluid reservoir (7,8,9) holding reagent fluid and at least one second fluid reservoir holding carrier fluid (10),
- sensing means (3) for collecting species from the medium, said sensing means having an inlet and an outlet,
- analysing means (22) for determining the concentration of the species in the medium,
- connecting means (21), comprising first connecting means for fluid connection between the first fluid reservoir and the analysing means, second connecting means for fluid connection between the second fluid reservoir and the inlet of the sensing means and third connecting means for fluid connection between the outlet of the sensing means and the analysing means,
- said first connecting means comprising at least one first flow restricting means (26,27,28) and said second connecting means comprising at least one second flow restricting means (30), **characterized in that**
a third fluid reservoir (11) holding priming fluid and fourth connecting means (32) for connection between said third fluid reservoir and the inlet of the sensing means are provided.

2. Micro-analysis system according to claim 1, wherein said fourth connecting means comprise at least one third flow restricting means.

3. Micro-analysis system according to claim 2, wherein said third flow restricting means exhibits less flow restriction than said second flow restricting means.

4. Micro-analysis system according to any of claims 1 to 3, wherein the volume of the third fluid reservoir is substantially equal to the total volume of said second and fourth connecting means and of said sensing means.

5. Micro-analysis system according to any of claim 2 to 4, wherein where said fourth connecting means communicate with said second connecting means downstream said second and third flow restricting means.

6. Micro-analysis system according to any of claims 1 to 5, further comprising means for pressurizing said fluid reservoirs.

7. Micro-analysis system according to claim 6, wherein at least parts of the walls of the fluid reservoirs are flexible.

8. Micro-analysis system according to claim 7, wherein the storage means further comprise a storage container with a fluid inlet communicating with said pressurizing means, said storage container delimiting a fluid chamber, within which the fluid reservoirs are arranged.

9. Micro-analysis system according to any of claims 6 to 8, wherein the pressurizing means is a constant pressure source.

10. Micro-analysis system according to any of claims 6 to 9, wherein said storage means, said connecting means, said analysing means and said pressurizing means are arranged within a common housing.

11. Micro-analysis system according to any of claims 1 to 10, wherein said medium is a human body fluid.

12. Micro-analysis system according to any of claims 1 to 11, wherein said sensing means is implanted in the body of a patient.

## Patentansprüche

1. Mikroanalysesystem zur Bestimmung der Konzentration einer Spezies in einem Medium, wobei das System Folgendes aufweist:
- Lagerungsmittel (6) zur Lagerung von Flüssigkeiten mit mindestens einem ersten Flüssigkeitsbehälter (7, 8, 9), der die Reagenzflüssigkeit enthält, und mindestens einem zweiten Flüssigkeitsbehälter (10), der die Trägerflüssigkeit enthält,
- Abtastmittel (3) zum Einsammeln von Spezies aus dem Medium, wobei die Abtastmittel einen Einlass und einen Auslass aufweisen,
- Analysemittel (22) zur Bestimmung der Konzentration der Spezies im Medium,
- Anschlussmittel (21), die erste Anschlussmittel zur Flüssigkeitsverbindung zwischen dem ersten Flüssigkeitsbehälter und den Analysemitteln, zweite Anschlussmittel zur Flüssigkeitsverbindung zwischen dem zweiten Flüssigkeitsbehälter und dem Einlass der Abtastmittel und dritte Anschlussmittel zur Flüssigkeitsverbindung zwischen dem Auslass der Alatastmittel und der Analysemittel aufweist,
- wobei die ersten Anschlussmittel mindestens erste Durchflussbegrenzungsmittel (26, 27, 28) und die zweiten Anschlussmittel mindestens zweite Durchflussbegrenzungsmittel (30) aufweisen,
**dadurch gekennzeichnet, dass** ein dritter Flüssigkeitsbehälter (11) mit Füllflüssigkeit und vierte Verbindungsmittel (32) zur Verbindung zwischen dem dritten Flüssigkeitsbehälter und dem Einlass der Abtastmittel vorgesehen sind.

2. Mikroanalysesystem nach Anspruch 1, wobei die vierten Anschlussmittel mindestens dritte Durchflussbegrenzungsmittel aufweisen.

3. Mikroanalysesystem nach Anspruch 2, wobei die dritten Durchflussbegrenzungsmittel eine kleinere Durchflussbegrenzung aufweisen als die zweiten Durchflussbegrenzungsmittel.

4. Mikroanalysesystem nach jedem der Ansprüche 1 bis 3,
wobei das Volumen des dritten Flüssigkeitsbehälters im Wesentlichen gleich des Gesamtvolumens der zweiten und vierten Anschlussmittel und der Abtastmittel ist.

5. Mikroanalysesystem nach jedem der Ansprüche 2 bis 4, wobei die vierten Anschlussmittel mit den zweiten Anschlussmitteln stromabwärts im Verhältnis zu den zweiten und dritten Durchflussbegrenzungsmitteln in Verbindung sind.

6. Mikroanalysesystem nach jedem der Ansprüche 1 bis 5, das zusätzlich Mittel aufweist, die die genannten Flüssigkeitsbehälter unter Druck setzen.

7. Mikroanalysesystem nach Anspruch 6, in dem zumindest Teile der wände der Flüssigkeitsbehälter flexibel sind.

8. Mikroanalysesystem nach Anspruch 7, wobei die Lagerungsmittel zusätzlich einen Lagerungsbehälter mit einer Flüssigkeitseinlassverbindung zu den genannten Druckmitteln aufweisen, wobei der Lagerungsbehälter eine Flüssigkeitskammer abgrenzt, in der die Flüssigkeitsbehälter angebracht sind.

9. Mikroanalysesystem nach jedem der Ansprüche 6 bis 8,
wobei die Druckmittel eine konstante Druckquelle sind.

10. Mikroanalysesystem nach jedem der Ansprüche 6 bis 9,
wobei die Lagerungsmittel, die Verbindungsmittel, die Analysemittel und die Druckmittel in einem gemeinsamen Gehäuse angebracht sind.

11. Mikroanalysesystem nach jedem der Ansprüche 1 bis 10,
wobei das Medium eine Körperflüssigkeit eines Menschen ist.

12. Mikroanalysesystem nach jedem der Ansprüche 1 bis 11,
wobei die Abtastmittel im Körper eines Patienten angebracht sind.

## Revendications

1. Système microanalytique permettant de déterminer la concentration d'un milieu en une espèce, ledit système comprenant
- un moyen de stockage (6) destiné au stockage de fluides, comprenant au moins un premier réservoir de fluide (7, 8, 9) contenant un fluide réactif et au moins un deuxième réservoir de fluide contenant un fluide support (10),
- un moyen de détection (3) pour le recueil d'espèces à partir du milieu, ledit moyen de détection comportant un orifice d'entrée et un orifice de sortie,
- un moyen d'analyse (22) permettant de déterminer la concentration du milieu en l'espèce,
- un moyen de raccordement (21) comportant un premier moyen de raccordement assurant une liaison fluidique entre le premier réservoir de fluide et le moyen d'analyse, un deuxième moyen de raccordement assurant une liaison fluidique entre le deuxième réservoir de fluide et l'orifice d'entrée du moyen de détection et un troisième moyen de raccordement assurant une liaison fluidique entre l'orifice de sortie du moyen de détection et le moyen d'analyse,
- ledit premier moyen de raccordement comprenant au moins un premier moyen de restriction d'écoulement (26, 27, 28) et ledit deuxième moyen de raccordement comprenant au moins un second moyen de restriction d'écoulement (30), **caractérisé en ce que**
sont prévus un troisième réservoir de fluide (11) contenant un fluide d'amorçage et un quatrième moyen de raccordement (32) assurant la liaison entre ledit troisième réservoir de fluide et l'orifice d'entrée du moyen de détection.

2. Système microanalytique selon la revendication 1, dans lequel ledit quatrième moyen de raccordement comprend au moins un troisième moyen de restriction d'écoulement.

3. Système microanalytique selon la revendication 2, dans lequel ledit troisième moyen de restriction d'écoulement présente une moindre restriction d'écoulement que ledit deuxième moyen de restriction d'écoulement.

4. Système microanalytique selon l'une quelconque des revendications 1 à 3, dans lequel le volume du troisième réservoir de fluide est sensiblement égal au volume total desdits deuxième et quatrième moyens de raccordement et dudit moyen de détection.

5. Système microanalytique selon l'une quelconque des revendications 2 à 4, dans lequel ledit quatrième moyen de raccordement communique avec ledit deuxième moyen de raccordement en aval desdits deuxième et troisième moyens de restriction d'écoulement.

6. Système microanalytique selon l'une quelconque des revendications 1 à 5, comprenant, en outre, un moyen de pressurisation desdits réservoirs de fluide.

7. Système microanalytique selon la revendication 6, dans lequel au moins des parties des parois des réservoirs de fluide sont souples.

8. Système microanalytique selon la revendication 7, dans lequel le moyen de stockage comprend, en outre, un réservoir de stockage comportant un orifice d'entrée de fluide en communication avec ledit moyen de pressurisation, ledit réservoir de stockage délimitant une chambre de fluide, dans laquelle sont disposés les réservoirs de fluide.

9. Système microanalytique selon l'une quelconque des revendications 6 à 8, dans lequel le moyen de pressurisation est constitué d'une source à pression constante.

10. Système microanalytique selon l'une quelconque des revendications 6 à 9, dans lequel ledit moyen de stockage, ledit moyen de raccordement, ledit moyen d'analyse et ledit moyen de pressurisation sont disposés dans une enceinte commune.

11. Système microanalytique selon l'une quelconque des revendications 1 à 10, dans lequel ledit milieu est un fluide corporel humain.

12. Système microanalytique selon l'une quelconque des revendications 1 à 11, dans lequel ledit moyen de détection est implanté dans l'organisme d'un patient.
